**Europäisches Patentamt**

**European Patent Office**

**Office européen des brevets**

(19)

(11) Veröffentlichungsnummer: **0 336 903 B1**

# EUROPÄISCHE PATENTSCHRIFT

(12)

(45) Veröffentlichungstag der Patentschrift:
27.12.91 Patentblatt 91/52

(51) Int. Cl.⁵ : **A61M 25/00**

(21) Anmeldenummer : **89810247.0**

(22) Anmeldetag : **04.04.89**

(54) Einführungsschleuse in einem Schlauchverbindungsstück für eine Katheteranordnung.

(30) Priorität : 07.04.88 CH 1278/88

(43) Veröffentlichungstag der Anmeldung :
11.10.89 Patentblatt 89/41

(45) Bekanntmachung des Hinweises auf die
Patenterteilung :
27.12.91 Patentblatt 91/52

(84) Benannte Vertragsstaaten :
CH DE FR GB IT LI

(56) Entgegenhaltungen :
EP-A- 0 190 388
DE-A- 3 624 745
US-A- 4 252 122

(73) Patentinhaber : SCHNEIDER (EUROPE) AG
Schärenmoosstrasse 115-117
CH-8052 Zürich (CH)

(72) Erfinder : Niederhauser, Werner
Bärenbohlstrasse 31
CH-8046 Zürich (CH)

(74) Vertreter : Groner, Manfred et al
Patentanwalts-Bureau Isler AG Postfach 6940
CH-8023 Zürich (CH)

## Beschreibung

Die Erfindung betrifft eine Einführungsschleuse für eine Katheteranordnung nach dem Oberbegriff des unabhängigen Patentanspruchs 1.

Eine Einführungsschleuse dieser Art ist durch die US-A-4,252,122 bekannt geworden. Diese weist einen zylindrischen Dichtungskörper auf, dessen Durchgang durch achsiales Quetschen mittels eines schraubbaren Deckels vermutlich im Durchmesser etwas geändert werden kann. Eine Anpassung an grosse Durchmesserunterschiede, wie sie beispielsweise beim Austausch eines Führungsdrahtes mit einem Durchmesser von 1 mm gegen einen Ballondilationskatheter mit einem Durchmesser von 6 mm notwendig ist, ist hier nicht möglich. Auch ist eine Manipulation an der Einführungsschleuse, die auch beim Quetschbinder nach der EP-A-0190388 notwendig wäre, nicht erwünscht.

Weiter ist durch die CH-A-654214 ein Schlauchverbindungsstück bekannt, in dem sich ein O-Ring aus Silikongummi befindet, durch den ein Trägerschlauch der Katheteranordnung flüssigkeitsdicht hindurchgesteckt ist. In einem weiteren Schlauchverbindungsstück ist ebenfalls eine radiale Dichtung angeordnet, die den länglichen Führungskörper, der wesentlich dünner ist als der Trägerschlauch, druckdicht umschliesst.

Bekannt ist ausserdem eine Einführungsschleuse für eine Katheteranordnung, die mehrere hintereinander angeordnete flache Dichtungskörper aufweist, die unterschiedliche Durchführungsöffnungen aufweisen. Jeder Dichtungskörper ist auf einen bestimmten Durchmesser abgestimmt, so dass jeweils nur einer dieser Dichtungskörper den in die Schleuse eingeführten länglichen Körper umschliesst.

In der Praxis erwiesen sich diese Schleusen oft als undicht. Auch setzen diese Dichtungskörper eine hohe Präzision und eine einwandfreie Montage voraus und sind deshalb vergleichsweise teuer.

Der Erfindung liegt die Aufgabe zugrunde, eine Einführungsschleuse der eingangs genannten Gattung zu schaffen, die auch im Durchmesser wesentlich unterschiedliche Körper zuverlässig und selbsttätig abdichtet.

Die Aufgabe wird durch die Erfindung gemäss des gekennzeichneten Teils des Anspruch 1 gelöst.

Der Dichtungskörper umschliesst hindurchtretende Körper mit einem Durchmesser von etwa 1 mm bis etwa 6 mm druckdicht und verbindet den Austritt von Gefässflüssigkeit bei der Einführung oder dem Wechsel eines Katheters. Da die erfindungsgemässe Einführungsschleuse lediglich einen Dichtungskörper aufweist, ist die Montage wesentlich einfacher und weniger fehleranfällig als die bisher bekannten Einführungsschleusen. Ein wesentlicher Vorteil ist, dass die Dichtung im Bereich von etwa 1 mm bis 6 mm stufenlos und selbstanpassend dichtet und zudem der durchtretende Katheter mit geringer Kraft in Längsrichtung bewegt werden kann. Die erfindungsgemässe Einführungsschleuse erleichtert somit die Handhabung der Katheteranordnung.

Durch die Weiterbildung nach Anspruch 2 ist es möglich, auch Katheter mit sehr grossen Durchmesserunterschieden zu verwenden, wobei bei leichter Verschiebbarkeit des Katheters die Druckdichtigkeit gewährleistet ist.

Vorteilhafte Halterungen des Dichtungskörpers werden durch die Weiterbildungen nach den Ansprüchen 3 und 4 realisiert.

Besonders gute Dichtungseigenschaften weist ein Dichtungskörper aus Silikongummi auf. Die Härte des Dichtungskörpers sollte weniger als 40 Shore betragen. Bewährt hat sich besonders ein Körper mit einer Härte von etwa 20 Shore.

Ein Ausführungsbeispiel der Erfindung wird nachfolgend anhand der Zeichnung näher erläutert. Es zeigen:

Fig. 1    eine Ansicht einer Katheteranordnung,
Fig. 2    einen Längsschnitt durch ein Schlauchverbindungsstück mit auseinandergezogenen Teilen,
Fig. 3a    und Fig. 3b einen Längsschnitt und eine Ansicht eines Dichtungskörpers mit einem eingeführten Führungsdraht, und
Fig. 3c    einen Längsschnitt durch einen Dichtungskörper mit einem eingeführten Katheter.

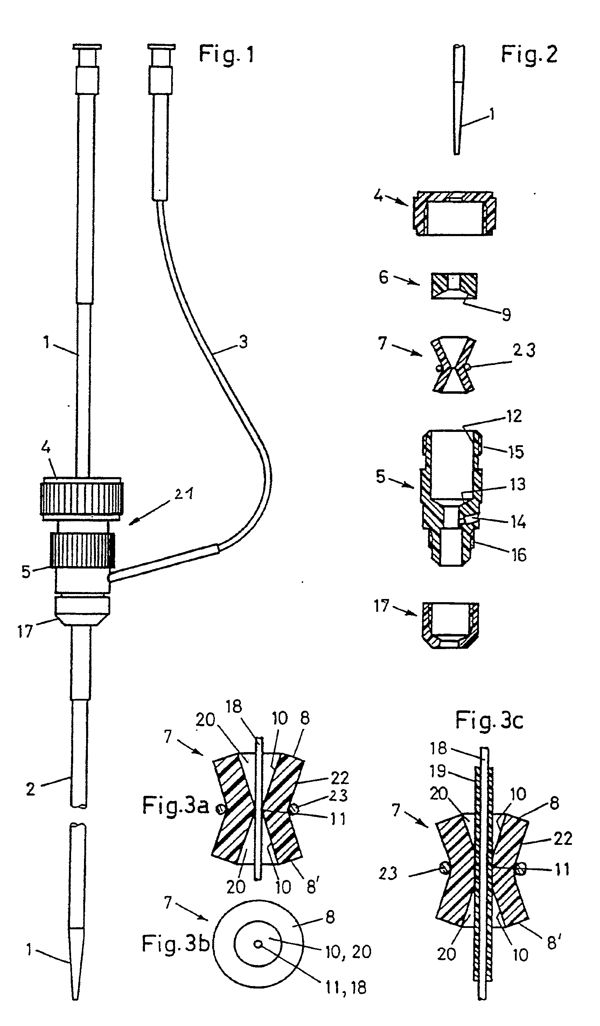

Wie die Figuren 1 und 2 zeigen, weist die Katheteranordnung ein Schlauchverbindungsstück 21 auf, an dem mittels einer Ueberwurfmutter 17 ein Einführungsschlauch 2, ein sogenannter Introducer, befestigt ist, der die Einführung eines an sich bekannten Führungsdrahtes 18 (Fig. 3a-3b) in das zu behandelnde Gefäss ermöglicht. Das Schlauchverbindungsstück 21 besitzt ein Gehäuse 5 mit einem im Querschnitt kreisrunden Durchgang 12, in den ein gummielastischer Dichtungskörper 7 und eine vergleichsweise harte Hülse 6 eingesetzt sind. In den Durchgang 12 mündet ein seitlicher Durchgang 14, in den ein Schlauch 3 eingesetzt ist, durch den beispielsweise ein Dilatationsballon mit einer Saug- und Druckpumpe verbunden werden kann.

Die Länge des Durchgangs 12 ist so bemessen, dass nach dem Aufschrauben eines Deckels 4 der Dichtungskörper 7 mit einer vorderen Fläche und einer hinteren Fläche 8' an entsprechenden ebenen Flächen 13 und 9 des Gehäuses bzw. der Hülse 6 anliegt. Wie die Fig. 3a deutlich zeigt, weist der Dichtungskörper 7 einen Durchgang 20 auf, der mittig eine Engstelle 11 mit einer Weite von etwa 1 mm besitzt, von der aus sich der Durchgang 20 nach beiden Seiten erweitert und der Kegelflächen 10 mit einem halben Oeffnungswinkel von etwa 20° bildet.

Die Engstelle 11 ist so bemessen, dass ein durch den Durchgang hindurchtretender dünner Führungskörper 18 vom Dichtungskörper 7 druckdicht umschlossen wird. In der Ruhestellung beträgt die Weite der Engstelle 11 etwa 1 mm, die im Fall der Verwendung noch dünnerer Führungskörper auch kleiner sein kann.

Die Mantelfläche 22 des Dichtungskörpers 7 ist etwa parallel zur Innenfläche 10, so dass der Körper 7 überall etwa die gleiche Wandstärke besitzt. Der Körper 7 besteht vorzugsweise aus Silikonkautschuk mit einer Härte unter 40 Shore, vorzugsweise etwa 20 Shore.

Ist gemäss Fig. 3c auf den Führungskörper 18 ein wesentlich dickerer Schlauch 19, beispielsweise mit einem Durchmesser von 6 mm, aufgeschoben, so ergibt sich eine vergleichsweise breite Kontaktfläche zwischen dem Dichtungskörper 7 und Schlauch 19 und eine Verschiebung insbesondere des mittigen Bereichs des Körpers 7 radial nach aussen. Da der Körper 7 lediglich vorne und hinten an den Flächen 8 und 8' abgestützt ist, kann er im mittigen Bereich ohne Erzeugung grosser radialer Kräfte und unter Vermeidung unkontrollierter Verformungen verdrängt werden. Der vergleichsweise dicke Schlauch 19 lässt sich deshalb mit geringer Reibung im Körper 7 in Längsrichtung verschieben.

Der Dichtungskörper 11 ist vorzugsweise spiegelsymmetrisch zu einer ihn halbierenden Querfläche und rotationssymmetrisch bezüglich seiner Längsachse. Er kann somit nicht verkehrt in das Gehäuse 5 eingesetzt werden. Auch übt er beim Hineinschieben und beim Herausziehen des Katheters gleich grosse Kräfte auf diesen aus.

Vorzugsweise ist ein gummielastischer Ring 23 mit kreisförmigem Profilquerschnitt um den gummielastischen Körper 7 gelegt, insbesondere wenn der Körper 7 aus Polyurethan ist. Versuche haben gezeigt, dass hierdurch eine Rissbildung am Körper 7 auch bei sehr starker Beanspruchung und einer radialen Dehnung von 400% vermieden werden kann. Der Ring 23 kann aus Gummi sein und umgreift den Körper 7 wie dargestellt an seiner engsten Stelle. Der Ring 23 unterstützt die sofortige Kontraktion des Körpers 7 bei einem Katheterwechsel.

Aus den obigen Angaben ergibt sich somit eine Einführungsschleuse, die bei einfachem Aufbau funktionssicher und auch bei einem Katheterwechsel dicht ist und eine einfache und sichere Steuerung des eingeführten Katheters erlaubt.

## Patentansprüche

1. Einführungsschleuse in einem Schlauchverbindungsstück (21) für eine Katheteranordnung, mit einer Dichtung, die das Schlauchverbindungsstück (21) druckdicht gegen einen vergleichsweise dünnen und länglichen Führungskörper (18) und gegebenenfalls auch gegen einen auf diesen aufgeschobenen Schlauch (19) abdichtet, wobei die Dichtung einen gummielastischen Körper (7) aufweist, der eine Durchtrittsöffnung (20) aufweist und der in Längsrichtung der Durchtrittsöffnung gesehen vorne und hinten am Schlauchverbindungsstück (21) abgestützt ist, dadurch gekennzeichnet, dass der gummielastische Körper (7) etwa mittig innen und aussen einen kleineren Durchmesser aufweist als weiter aussen, und dass zwischen der Mantelfläche des gummielastischen Körpers (7) und dem Gehäuse (5) des Schlauchverbindungsstückes (21) ein Zwischenraum vorgesehen ist, in welchen der gummielastische Körper (7) zur Selbstanpassung an den Aussendurchmesser des Führungskörpers (18) bzw. des Schlauches (19) im Bereich der engsten Stelle der Durchtrittsöffnung (20) radial ausweichen kann.

2. Einführungsschleuse nach Anspruch 1, dadurch gekennzeichnet, dass der gummielastische Körper eine Mantelfläche (22) aufweist, die im wesentlichen parallel zu seiner Innenfläche (10) ist.

3. Einführungsschleuse nach Anspruch 1 oder 2, dadurch gekennzeichnet, dass an das Schlauchverbindungsstück (21) anliegende Flächen (8, 8') des gummielastischen Körpers (7) eben sind und etwa rechtwinklig zur Innenfläche (10) verlaufen.

4.. Einführungsschleuse nach einem der Ansprüche 1 bis 3, dadurch gekennzeichnet, dass eine der anliegenden Flächen (8) an einer in das Schlauchverbindungsstück eingesetzten Hülse (6) anliegt.

5. Einführungsschleuse nach einem der Ansprüche 1 bis 5, dadurch gekennzeichnet, dass der gummielastische Körper (7) eine Härte unter etwa 40 Shore aufweist.

6. Einführungsschleuse nach anspruch 5, dadurch gekennzeichnet, dass die Härte des gummielastischen Körpers etwa 20 Shore beträgt.

7. Einführungsschleuse nach einem der Ansprüche 1 bis 6, gekennzeichnet durch einen den gummielastischen Körper (7) umgreifenden Ring (23) aus gummielastischem Werkstoff.

8. Einführungsschleuse nach Anspruch 7, dadurch gekennzeichnet, dass der gummielastische Körper (7) aus Polyurethan besteht.

9. Einführungsschleuse nach Anspruch 7 oder 8, dadurch gekennzeichnet, dass der gummielastische Ring (23) mittig umfasst.

10. Einführungsschleuse nach Anspruch 1, dadurch gekennzeichnet, dass der gummielastische Körper (7) an beiden Enden eine Durchtrittsöffnung (20) aufweist.

## Claims

1. An introduction lock in a hose connector (21) for a catheter arrangement, comprising a seal which seals the hose connector (21) hermetically from a comparatively thin and elongate guide member (18) and, if required, also from a hose (19) pushed over the guide member (18), the seal comprising an elastomeric member (7) having a passage aperture (20) and supported on the hose connector (21) front and rear as considered in the longitudinal direction of the passage aperture, characterised in that the elastomeric member (7) has substantially centrally internally and externally a smaller diameter than farther out, and in that there is provided between the outer surface of the elastomeric member (7) and the housing (5) of the hose connector (21) an intermediate space into which the elastomeric member (7) can radially yield for automatic adaptation to the outside diameter of the guide member (18) or of the hose (19) in the region of the narrowest point of the passage aperture (20).

2. An introduction lock according to claim 1, characterised in that the elastomeric member has an outer surface (22) which is substantially parallel to its inner surface (10).

3. An introduction lock according to claim 1 or 2, characterised in that surfaces (8, 8') of the elastomeric member (7) which abut the hose connector (21) are flat and extend substantially at right angles to the inner surface (10).

4. An introduction lock according to any one of claims 1 to 3, characterised in that one of the abutting surfaces (8) abuts a sleeve (6) inserted into the hose connector.

5. An introduction lock according to any one of claims 1 to 5, characterised in that the elastomeric member (7) has a hardness below approximately 40 Shore.

6. An introduction lock according to claim 5, characterised in that the hardness of the elastomeric member is about 20 Shore.

7. An introduction lock according to any one of claims 1 to 6, characterised by a ring (23) of elastomeric material engaging around the elastomeric member (7).

8. An introduction lock according to claim 7, characterised in that the elastomeric member (7) consists of polyurethane.

9. An introduction lock according to claim 7 or 8, characterised in that the elastomeric ring (23) engages centrally.

10. An introduction lock according to claim 1, characterised in that the elastomeric member (7) has a passage aperture (20) at both ends.

## Revendications

1. Ecluse guide dans un raccord pour tuyaux (21) destinée à un système de cathéter, avec un élément d'étanchéité qui rend le raccord pour tuyaux (21) étanche à la pression vis-à-vis d'un corps de guidage (18) comparativement mince et allongé et éventuellement, également vis-à-vis d'un tuyau flexible (19) monté sur celui-ci, l'élément d'étanchéité présentant un corps élastique en caoutchouc (7) qui comporte une ouverture de passage (20) et qui s'appuie vu dans la direction longitudinale de l'ouverture de passage à l'avant et à l'arrière sur le raccord pour tuyaux (21), caractérisée en ce que le corps en caoutchouc élastique (7) présente sensiblement centralement à l'intérieur et à l'extérieur un plus petit diamètre qui va en s'élargissant vers l'extérieur, et en ce qu'entre la surface d'enveloppe du corps en caoutchouc élastique (7) et le logement (5) du raccord pour tuyaux (21) est prévu un espace intermédiaire dans lequel le corps en caoutchouc élastique peut se dégager radialement pour l'auto-adaptation au diamètre extérieur du corps de guidage (18) ou du tuyau flexible (19) au niveau de l'endroit le plus étroit de l'ouverture de passage (20).

2. Ecluse guide selon la revendication 1, caractérisée en ce que le corps en caoutchouc élastique présente une surface d'enveloppe (22) qui est essentiellement parallèle à une surface interne (10).

3. Ecluse guide selon la revendication 1 ou 2, caractérisée en ce que sur le raccord pour tuyaux (21) les surfaces attenantes (8, 8') du corps en caoutchouc élastique (7) sont planes et s'étendent sensiblement à angle droit par rapport à la surface interne (10).

4. Ecluse guide selon l'une des revendications 1 à 3, caractérisée en ce que l'une des surfaces attenantes (8) est contiguë à une douille (6) introduite dans le raccord pour tuyaux.

4

5. Ecluse guide selon l'une des revendications 1 à 5, caractérisée en ce que le corps en caoutchouc élastique (7) possède une dureté inférieure à sensiblement 40 Shore.

6. Ecluse guide selon la revendication 5, caractérisée en ce que le corps en caoutchouc élastique a une dureté sensiblement égale à 20 Shore.

7. Ecluse guide selon l'une des revendications 1 à 6, caractérisée par un anneau (23) enserrant le corps en caoutchouc élastique (7), anneau réalisé en un matériau en caoutchouc élastique.

8. Ecluse guide selon la revendication 7, caractérisée en ce que le corps en caoutchouc élastique (7) est en polyuréthane.

9. Ecluse guide selon la revendication 7 ou 8, caractérisée en ce que l'anneau en caoutchouc élastique (23) est positionné centralement.

10. Ecluse guide selon la revendication 1, caractérisée en ce que le corps en caoutchouc élastique (7) présente une ouverture de passage (20) sur les deux extrémités.

Fig.1

Fig.2

Fig.3a

Fig.3b

Fig.3c